(19) Europäisches Patentamt
European Patent Office
Office européen des brevets

(11) Veröffentlichungsnummer: **0 199 211**
**A2**

(12) **EUROPÄISCHE PATENTANMELDUNG**

(21) Anmeldenummer: 86104971.6

(22) Anmeldetag: 11.04.86

(51) Int. Cl.[4]: C 09 K 19/40
C 09 K 19/34, C 09 K 19/32
C 07 C 101/12, C 07 C 87/30
C 07 C 149/273, C 07 C 149/20
C 07 C 69/00, C 07 C 143/11
C 07 D 213/06

(30) Priorität: 17.04.85 DE 3513795

(43) Veröffentlichungstag der Anmeldung:
29.10.86 Patentblatt 86/44

(84) Benannte Vertragsstaaten:
CH DE FR GB IT LI NL

(71) Anmelder: BASF Aktiengesellschaft
Carl-Bosch-Strasse 38
D-6700 Ludwigshafen(DE)

(72) Erfinder: Ringsdorf, Helmut, Prof. Dr.
Kehlweg 41
D-6500 Mainz-Gonsenheim(DE)

(72) Erfinder: Griffith, Renate
Freiherr von Wallbrunn Strasse 17
D-6501 Partenheim(DE)

(72) Erfinder: Vierengel, Anita
Hochheimer Strasse 58
D-6502 Mainz-Kostheim(DE)

(72) Erfinder: Neumann, Peter, Dr.
Franz-Schubert-Strasse 1
D-6908 Wiesloch(DE)

(72) Erfinder: Hisgen, Bernd, Dr.
Goethestrasse 6
D-6703 Limburgerhof(DE)

(72) Erfinder: Horn, Dieter, Dr.
Schroederstrasse 69
D-6900 Heidelberg(DE)

(54) **Amphiphile flüssigkristalline Verbindungen.**

(57) Amphiphile flüssigkristalline Verbindungen der allgemeinen Formel

$$D-[Z-A-K]_i$$

in der
D ein Cyclohexanring oder ein planares aromatisches System,
Z eine direkte Bindung oder ein Rest der Formel $-O-$, $-S-$, $-SO_2-$, $-CO-$, $-O-CO-$ oder $-CO-O$,
A gegebenenfalls durch einen Rest Z unterbrochenes Alkylen und
K ein anionischer oder kationischer Rest sind, wobei
i die Zahlen 4, 5 oder 6 bedeutet, wenn D ein Benzolrest ist, oder $> 2$ ist, wenn D für einen Cyclohexanring oder ein planares aromatisches System, ausgenommen des Benzols steht.

Die neuen Verbindungen bilden in wässriger oder wässrig-alkoholischen Lösungen lyotrop nematische Phasen. Sie sind dementsprechend als Komplexbildner, Tenside, Schmierstoffadditive oder Strömungsbeschleuniger verwendbar.

EP 0 199 211 A2

Amphiphile flüssigkristalline Verbindungen

Bekannt sind micellbildende Systeme, deren charakteristische Eigenschaften zu vielseitigen Anwendungen in Technik und Forschung geführt haben (B. Lindmann, H. Wennerstrom in: Topics in Current Chemistry 87, S.1 ff). Weiterhin bekannt sind lyotrope, flüssigkristalline Phasen bildende Systeme (P. A. Winsor, Chem. Rev. 68, 1 (1968)).

Seit einiger Zeit sind auch Systeme bekannt, die im Grenzbereich zwischen isotroper Kugelmicellösung und lyotropen Flüssigkristallphasen formanisotrope zylinder- oder scheibchenförmige Micellen bilden (H. Hoffmann, et. al., Ber. Bunsenges. Phys. Chem. 85, 255 (1981)).

Es wurde gefunden, daß amphiphile Verbindungen der unten angegebenen Formel (I), die ionische Kopfgruppen über Spacer an einen scheibenförmigen hydrophoben Kern gebunden enthalten, bevorzugt anisotrope Micellen und entsprechende lyotrope Phasen bilden.

Dementsprechend betrifft die Erfindung amphiphile flüssigkristalline Verbindungen der allgemeinen Formel

$$D\!-\!\left[-Z-A-K\right]_i \quad ,$$

in der

D ein Cyclohexanring oder ein planares aromatisches System,

Z eine direkte Bindung oder ein Rest der Formel $-O-$, $-S-$, $-SO_2-$, $-CO-$, $-O-CO-$ oder $-CO-O-$,

A gegebenenfalls durch einen Rest Z unterbrochenes Alkylen und

K ein anionischer oder kationischer Rest sind, wobei

i die Zahlen 4, 5 oder 6 bedeutet, wenn D ein Benzolrest ist, oder > 2 ist, wenn D für einen Cyclohexanring oder ein planares aromatisches System, ausgenommen des Benzols steht.

Reste D leiten sich z.B. von Verbindungen der Formeln

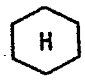 , 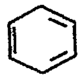 , 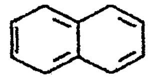 , 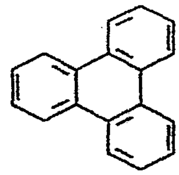 oder 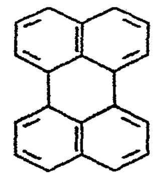

ab. Bevorzugt leitet D sich vom Benzol- oder Cyclohexankern ab, wobei i dann 4, 5 oder 6 ist, wenn D sich vom Benzol ableitet. Leitet sich D vom Cyclohexan oder von kondensierten aromatischen Systemen wie Naphthalin, Triphenylen oder Perylen ab, kann i 3, 4, 5, 6, 7, 8 oder 9 sein.

Ist i kleiner als die Zahl der maximal an D zur Verfügung stehenden Substituenten, so kann der Kern weitere, nicht ionische Reste tragen, z.B. Halogenatome oder Methylgruppen.

Reste K sind z.B.:

$-SO_3^{\ominus}\,Me^{\oplus}$, $-N\left[CH_2CH_2SO_3^{\ominus}\,Me^{\oplus}\right]_2$, $-COO^{\ominus}\,Me^{\oplus}$, $-N\left[-\left[CH_2\right]_r-COO^{\ominus}\,Me^{\oplus}\right]_2$,

$-OCH_2COO^{\ominus}\,Me^{\oplus}$, $-CH\left[COO^{\ominus}\,Me^{\oplus}\right]_2$, $-N(R)_3\,X^{\ominus}$ oder 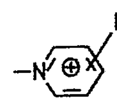 $X^{\ominus}$ , wobei

r für die Zahl 1 oder 2, R für Methyl oder Ethyl, $Me^{\oplus}$ für ein Proton, ein Metallion, $NH_4^{\oplus}$ oder $N(CH_3)_4^{\oplus}$ und $X^{\ominus}$ für ein Anion stehen.

Als Metallionen kommen für $Me^{\oplus}$ beispielsweise in Betracht:

$Li^{\oplus}$, $Na^{\oplus}$, $K^{\oplus}$, $Rb^{\oplus}$, $Cs^{\oplus}$, $\frac{Mg^{2\oplus}}{2}$, $\frac{Ca^{2\oplus}}{2}$, $\frac{Zn^{2\oplus}}{2}$, $\frac{Mn^{2\oplus}}{2}$, $\frac{Fe^{2\oplus}}{2}$, $\frac{Ni^{2\oplus}}{2}$ oder $Ag^{\oplus}$.

Als Anionen $X^{\ominus}$ sind beispielhaft zu nennen: Halogenidionen, vorzugsweise $Cl^{\ominus}$ und $Br^{\ominus}$, $CN^{\ominus}$, $SCN^{\ominus}$, $HSO_4^{\ominus}$, $BF_4^{\ominus}$, $H_3CCOO^{\ominus}$, $C_6H_5SO_3^{\ominus}$ oder $H_3C-C_6H_4-SO_3^{\ominus}$.

Die polaren Kopfgruppen sind über einen Spacer der Formel

-Z-A-an D gebunden, wobei Z und A die oben angegebene Bedeutung haben. Der Spacer -Z-A- sollte in der linearen Kette mindestens 6 Atome aufweisen.
Z steht für eine chemische Bindung oder einen Rest der Formel $-O-$, $-S-$,

$-SO_2-$, $-CO-$, $-O-CO-$ oder $-CO-O-$, vorzugsweise für $-O-$, $-S-$, $-O-CO-$ oder $-CO-O-$.

Als Reste A sind z.B. zu nennen:

$\left[CH_2\right]_n$ mit n = 3 bis 20, vorzugsweise 6 bis 18,

z.B. $\left[CH_2\right]_6$, $\left[CH_2\right]_7$, $\left[CH_2\right]_8$, $\left[CH_2\right]_9$, $\left[CH_2\right]_{10}$, $\left[CH_2\right]_{11}$, $\left[CH_2\right]_{12}$,

$\left[CH_2\right]_{14}$, $\left[CH_2\right]_{16}$ und $\left[CH_2\right]_{18}$

oder die entsprechenden Alkylenreste mit

$-(\overset{CH_3}{\overset{|}{C}H}-CH_2)_m-$ oder $-(CH_2-\overset{CH_3}{\overset{|}{C}H})_m-$, wobei m = 3 bis 10 ist.

Ferner sind die durch Z unterbrochenen Alkylenreste zu nennen, die vorzugsweise der Formel

$$-(CH_2)_p-Z^1-(CH_2)_q-$$

entsprechen, in der

p die Zahlen 1, 2 oder 3 und

q die Zahlen 2 bis 20 sind und

$Z^1$ $-O-$, $-S-$, $-O-CO-$ oder $-CO-O-$ bedeuten.

Vorzugsweise ist p gleich 1.

Einzelne derartige Reste sind z.B.:

$-CH_2-O-(CH_2)_3-$, $-CH_2-O-(CH_2)_4-$, $-CH_2-O-(CH_2)_5-$, $-CH_2-O-(CH_2)_6-$,

$-CH_2-O-(CH_2)_7-$, $-CH_2-O-(CH_2)_8-$, $-CH_2-O-(CH_2)_9-$, $-CH_2-O-(CH_2)_{10}-$,

$-CH_2-O-(CH_2)_{11}-$, $-CH_2-O-(CH_2)_{12}-$, $-CH_2-O-(CH_2)_{13}-$, $-CH_2-O-(CH_2)_{14}-$,

$-CH_2-O-(CH_2)_{15}-$, $-CH_2-O-(CH_2)_{16}-$, $-CH_2-O-(CH_2)_{17}-$, $-CH_2-O-(CH_2)_{18}-$,

$-CH_2-O-(CH_2)_{19}-$, $-CH_2-O-(CH_2)_{20}-$,

$-CH_2-S-(CH_2)_3-$, $-CH_2-S-(CH_2)_4-$, $-CH_2-S-(CH_2)_5-$, $-CH_2-S-(CH_2)_6-$, $-CH_2-S-(CH_2)_7-$, $-CH_2-S-(CH_2)_8-$, $-CH_2-S-(CH_2)_9-$, $-CH_2-S-(CH_2)_{10}-$, $-CH_2-S-(CH_2)_{11}-$, $-CH_2-S-(CH_2)_{12}-$, $-CH_2-S-(CH_2)_{13}-$, $-CH_2-S-(CH_2)_{14}-$, $-CH_2-S-(CH_2)_{15}-$, $-CH_2-S-(CH_2)_{16}-$, $-CH_2-S-(CH_2)_{17}-$, $-CH_2-S-(CH_2)_{18}-$, $-CH_2-S-(CH_2)_{19}-$, $-CH_2-S-(CH_2)_{20}-$,

$-CH_2-SO_2-(CH_2)_3-$, $-CH_2-SO_2-(CH_2)_4-$, $-CH_2-SO_2-(CH_2)_5-$, $-CH_2-SO_2-(CH_2)_6-$, $-CH_2-SO_2-(CH_2)_7-$, $-CH_2-SO_2-(CH_2)_8-$, $-CH_2-SO_2-(CH_2)_9-$, $-CH_2-SO_2-(CH_2)_{10}-$, $-CH_2-SO_2-(CH_2)_{11}-$, $-CH_2-SO_2-(CH_2)_{12}-$, $-CH_2-SO_2-(CH_2)_{13}-$, $-CH_2-SO_2-(CH_2)_{14}-$ $-CH_2-SO_2-(CH_2)_{15}-$, $-CH_2-SO_2-(CH_2)_{16}-$, $-CH_2-SO_2-(CH_2)_{17}-$, $-CH_2-SO_2-(CH_2)_{18}-$ $-CH_2-SO_2-(CH_2)_{19}-$, $-CH_2-SO_2-(CH_2)_{20}-$,

$-CH_2-CO-(CH_2)_3-$, $-CH_2-CO-(CH_2)_4-$, $-CH_2-CO-(CH_2)_5-$, $-CH_2-CO-(CH_2)_6-$, $-CH_2-CO-(CH_2)_7-$, $-CH_2-CO-(CH_2)_8-$, $-CH_2-CO-(CH_2)_9-$, $-CH_2-CO-(CH_2)_{10}-$, $-CH_2-CO-(CH_2)_{11}-$, $-CH_2-CO-(CH_2)_{12}-$, $-CH_2-CO-(CH_2)_{13}-$, $-CH_2-CO-(CH_2)_{14}-$ $-CH_2-CO-(CH_2)_{15}-$, $-CH_2-CO-(CH_2)_{16}-$, $-CH_2-CO-(CH_2)_{17}-$, $-CH_2-CO-(CH_2)_{18}-$ $-CH_2-CO-(CH_2)_{19}-$, $-CH_2-CO-(CH_2)_{20}-$,

$-CH_2-O-CO-(CH_2)_3-$, $-CH_2-O-CO-(CH_2)_4-$, $-CH_2-O-CO-(CH_2)_5-$, $-CH_2-O-CO-(CH_2)_6-$, $-CH_2-O-CO-(CH_2)_7-$, $-CH_2-O-CO-(CH_2)_8-$, $-CH_2-O-CO-(CH_2)_9-$, $-CH_2-O-CO-(CH_2)_{10}-$, $-CH_2-O-CO-(CH_2)_{11}-$, $-CH_2-O-CO-(CH_2)_{12}-$, $-CH_2-O-CO-(CH_2)_{13}-$, $-CH_2-O-CO-(CH_2)_{14}-$, $-CH_2-O-CO-(CH_2)_{15}-$, $-CH_2-O-CO-(CH_2)_{16}-$, $-CH_2-O-CO-(CH_2)_{17}-$, $-CH_2-O-CO-(CH_2)_{18}-$, $-CH_2-O-CO-(CH_2)_{19}-$, $-CH_2-O-CO-(CH_2)_{20}-$,

$-CH_2-CO-O-(CH_2)_3-$, $-CH_2-CO-O-(CH_2)_4-$, $-CH_2-CO-O-(CH_2)_5-$, $-CH_2-CO-O-(CH_2)_6-$, $-CH_2-CO-O-(CH_2)_7-$, $-CH_2-CO-O-(CH_2)_8-$, $-CH_2-CO-O-(CH_2)_9-$, $-CH_2-CO-O-(CH_2)_{10}-$, $-CH_2-CO-O-(CH_2)_{11}-$,

$-CH_2-CO-O-(CH_2)_{12}-$, $-CH_2-CO-O-(CH_2)_{13}-$, $-CH_2-CO-O-(CH_2)_{14}-$, $-CH_2-CO-O-(CH_2)_{15}-$, $-CH_2-CO-O-(CH_2)_{16}-$, $-CH_2-CO-O-(CH_2)_{17}-$, $-CH_2-CO-O-(CH_2)_{18}-$, $-CH_2-CO-O-(CH_2)_{19}-$, $-CH_2-CO-O-(CH_2)_{20}-$,

$-(CH_2)_2-O-(CH_2)_2-$, $-(CH_2)_2-O-(CH_2)_3-$, $-(CH_2)_2-O-(CH_2)_4-$, $-(CH_2)_2-O-(CH_2)_5-$, $-(CH_2)_2-O-(CH_2)_6-$, $-(CH_2)_2-O-(CH_2)_7-$, $-(CH_2)_2-O-(CH_2)_8-$, $-(CH_2)_2-O-(CH_2)_9-$, $-(CH_2)_2-O-(CH_2)_{10}-$, $-(CH_2)_2-O-(CH_2)_{11}-$, $-(CH_2)_2-O-(CH_2)_{12}-$, $-(CH_2)_2-O-(CH_2)_{13}-$, $-(CH_2)_2-O-(CH_2)_{14}-$, $-(CH_2)_2-O-(CH_2)_{15}-$, $-(CH_2)_2-O-(CH_2)_{16}-$, $-(CH_2)_2-O-(CH_2)_{17}-$, $-(CH_2)_2-O-(CH_2)_{18}-$, $-(CH_2)_2-O-(CH_2)_{19}-$, $-(CH_2)_2-O-(CH_2)_{20}-$,

$-(CH_2)_2-S-(CH_2)_2-$, $-(CH_2)_2-S-(CH_2)_3-$, $-(CH_2)_2-S-(CH_2)_4-$, $-(CH_2)_2-S-(CH_2)_5-$, $-(CH_2)_2-S-(CH_2)_6-$, $-(CH_2)_2-S-(CH_2)_7-$, $-(CH_2)_2-S-(CH_2)_8-$, $-(CH_2)_2-S-(CH_2)_9-$, $-(CH_2)_2-S-(CH_2)_{10}-$, $-(CH_2)_2-S-(CH_2)_{11}-$, $-(CH_2)_2-S-(CH_2)_{12}-$, $-(CH_2)_2-S-(CH_2)_{13}-$, $-(CH_2)_2-S-(CH_2)_{14}-$, $-(CH_2)_2-S-(CH_2)_{15}-$, $-(CH_2)_2-S-(CH_2)_{16}-$, $-(CH_2)_2-S-(CH_2)_{17}-$, $-(CH_2)_2-S-(CH_2)_{18}-$, $-(CH_2)_2-S-(CH_2)_{19}-$, $-(CH_2)_2-S-(CH_2)_{20}-$,

$-(CH_2)_2-SO_2-(CH_2)_2-$, $-(CH_2)_2-SO_2-(CH_2)_3-$, $-(CH_2)_2-SO_2-(CH_2)_4-$, $-(CH_2)_2-SO_2-(CH_2)_5-$, $-(CH_2)_2-SO_2-(CH_2)_6-$, $-(CH_2)_2-SO_2-(CH_2)_7-$, $-(CH_2)_2-SO_2-(CH_2)_8-$, $-(CH_2)_2-SO_2-(CH_2)_9-$, $-(CH_2)_2-SO_2-(CH_2)_{10}-$, $-(CH_2)_2-SO_2-(CH_2)_{11}-$, $-(CH_2)_2-SO_2-(CH_2)_{12}-$, $-(CH_2)_2-SO_2-(CH_2)_{13}-$, $-(CH_2)_2-SO_2-(CH_2)_{14}-$, $-(CH_2)_2-SO_2-(CH_2)_{15}-$, $-(CH_2)_2-SO_2-(CH_2)_{16}-$, $-(CH_2)_2-SO_2-(CH_2)_{17}-$, $-(CH_2)_2-SO_2-(CH_2)_{18}-$, $-(CH_2)_2-SO_2-(CH_2)_{19}-$, $-(CH_2)_2-SO_2-(CH_2)_{20}-$,

$-(CH_2)_2-CO-(CH_2)_2-$, $-(CH_2)_2-CO-(CH_2)_3-$, $-(CH_2)_2-CO-(CH_2)_4-$, $-(CH_2)_2-CO-(CH_2)_5-$, $-(CH_2)_2-CO-(CH_2)_6-$, $-(CH_2)_2-CO-(CH_2)_7-$, $-(CH_2)_2-CO-(CH_2)_8-$, $-(CH_2)_2-CO-(CH_2)_9-$, $-(CH_2)_2-CO-(CH_2)_{10}-$, $-(CH_2)_2-CO-(CH_2)_{11}-$, $-(CH_2)_2-CO-(CH_2)_{12}-$, $-(CH_2)_2-CO-(CH_2)_{13}-$, $-(CH_2)_2-CO-(CH_2)_{14}-$, $-(CH_2)_2-CO-(CH_2)_{15}-$, $-(CH_2)_2-CO-(CH_2)_{16}-$, $-(CH_2)_2-CO-(CH_2)_{17}-$, $-(CH_2)_2-CO-(CH_2)_{18}-$, $-(CH_2)_2-CO-(CH_2)_{19}-$, $-(CH_2)_2-CO-(CH_2)_{20}-$,

$-(CH_2)_2-O-CO-(CH_2)_2-$, $-(CH_2)_2-O-CO-(CH_2)_3-$, $-(CH_2)_2-O-CO-(CH_2)_4-$, $-(CH_2)_2-O-CO-(CH_2)_5-$, $-(CH_2)_2-O-CO-(CH_2)_6-$, $-(CH_2)_2-O-CO-(CH_2)_7-$, $-(CH_2)_2-O-CO-(CH_2)_8-$, $-(CH_2)_2-O-CO-(CH_2)_9-$, $-(CH_2)_2-O-CO-(CH_2)_{10}-$, $-(CH_2)_2-O-CO-(CH_2)_{11}-$, $-(CH_2)_2-O-CO-(CH_2)_{12}-$, $-(CH_2)_2-O-CO-(CH_2)_{13}-$, $-(CH_2)_2-O-CO-(CH_2)_{14}-$, $-(CH_2)_2-O-CO-(CH_2)_{15}-$, $-(CH_2)_2-O-CO-(CH_2)_{16}-$, $-(CH_2)_2-O-CO-(CH_2)_{17}-$, $-(CH_2)_2-O-CO-(CH_2)_{18}-$, $-(CH_2)_2-O-CO-(CH_2)_{19}-$, $-(CH_2)_2-O-CO-(CH_2)_{20}-$,

$-(CH_2)_2-CO-O-(CH_2)_2-$, $-(CH_2)_2-CO-O-(CH_2)_3-$, $-(CH_2)_2-CO-O-(CH_2)_4-$, $-(CH_2)_2-CO-O-(CH_2)_5-$, $-(CH_2)_2-CO-O-(CH_2)_6-$, $-(CH_2)_2-CO-O-(CH_2)_7-$, $-(CH_2)_2-CO-O-(CH_2)_8-$, $-(CH_2)_2-CO-O-(CH_2)_9-$, $-(CH_2)_2-CO-O-(CH_2)_{10}-$, $-(CH_2)_2-CO-O-(CH_2)_{11}-$, $-(CH_2)_2-CO-O-(CH_2)_{12}-$, $-(CH_2)_2-CO-O-(CH_2)_{13}-$, $-(CH_2)_2-CO-O-(CH_2)_{14}-$, $-(CH_2)_2-CO-O-(CH_2)_{15}-$, $-(CH_2)_2-CO-O-(CH_2)_{16}-$, $-(CH_2)_2-CO-O-(CH_2)17-$, $-(CH_2)_2-CO-O-(CH_2)_{18}-$, $-(CH_2)_2-CO-O-(CH_2)_{19}-$, $-(CH_2)_2-CO-O-(CH_2)_{20}-$,

$-(CH_2)_3-O-(CH_2)_2-$, $-(CH_2)_3-O-(CH_2)_3-$, $-(CH_2)_3-O-(CH_2)_4-$, $-(CH_2)_3-O-(CH_2)_5-$, $-(CH_2)_3-O-(CH_2)_6-$, $-(CH_2)_3-O-(CH_2)_7-$, $-(CH_2)_3-O-(CH_2)_8-$, $-(CH_2)_3-O-(CH_2)_9-$, $-(CH_2)_3-O-(CH_2)_{10}-$,

$-(CH_2)_3-O-(CH_2)_{11}-$, $-(CH_2)_3-O-(CH_2)_{12}-$, $-(CH_2)_3-O-(CH_2)_{13}-$, $-(CH_2)_3-O-(CH_2)_{14}-$, $-(CH_2)_3-O-(CH_2)_{15}-$, $-(CH_2)_3-O-(CH_2)_{16}-$, $-(CH_2)_3-O-(CH_2)_{17}-$, $-(CH_2)_3-O-(CH_2)_{18}-$, $-(CH_2)_3-O-(CH_2)_{19}-$, $-(CH_2)_3-O-(CH_2)_{20}-$,

$-(CH_2)_3-S-(CH_2)_2-$, $-(CH_2)_3-S-(CH_2)_3-$, $-(CH_2)_3-S-(CH_2)_4-$, $-(CH_2)_3-S-(CH_2)_5-$, $-(CH_2)_3-S-(CH_2)_6-$, $-(CH_2)_3-S-(CH_2)_7-$, $-(CH_2)_3-S-(CH_2)_8-$, $-(CH_2)_3-S-(CH_2)_9-$, $-(CH_2)_3-S-(CH_2)_{10}-$, $-(CH_2)_3-S-(CH_2)_{11}-$, $-(CH_2)_3-S-(CH_2)_{12}-$, $-(CH_2)_3-S-(CH_2)_{13}-$, $-(CH_2)_3-S-(CH_2)_{14}-$, $-(CH_2)_3-S-(CH_2)_{15}-$, $-(CH_2)_3-S-(CH_2)_{16}-$, $-(CH_2)_3-S-(CH_2)_{17}-$, $-(CH_2)_3-S-(CH_2)_{18}-$, $-(CH_2)_3-S-(CH_2)_{19}-$, $-(CH_2)_3-S-(CH_2)_{20}-$,

$-(CH_2)_3-SO_2-(CH_2)_2-$, $-(CH_2)_3-SO_2-(CH_2)_3-$, $-(CH_2)_3-SO_2-(CH_2)_4-$, $-(CH_2)_3-SO_2-(CH_2)_5-$, $-(CH_2)_3-SO_2-(CH_2)_6-$, $-(CH_2)_3-SO_2-(CH_2)_7-$, $-(CH_2)_3-SO_2-(CH_2)_8-$, $-(CH_2)_3-SO_2-(CH_2)_9-$, $-(CH_2)_3-SO_2-(CH_2)_{10}-$, $-(CH_2)_3-SO_2-(CH_2)_{11}-$, $-(CH_2)_3-SO_2-(CH_2)_{12}-$, $-(CH_2)_3-SO_2-(CH_2)_{13}-$, $-(CH_2)_3-SO_2-(CH_2)_{14}-$, $-(CH_2)_3-SO_2-(CH_2)_{15}-$, $-(CH_2)_3-SO_2-(CH_2)_{16}-$, $-(CH_2)_3-SO_2-(CH_2)_{17}-$, $-(CH_2)_3-SO_2-(CH_2)_{18}-$, $-(CH_2)_3-SO_2-(CH_2)_{19}-$, $-(CH_2)_3-SO_2-(CH_2)_{20}-$,

$-(CH_2)_3-CO-(CH_2)_2-$, $-(CH_2)_3-CO-(CH_2)_3-$, $-(CH_2)_3-CO-(CH_2)_4-$, $-(CH_2)_3-CO-(CH_2)_5-$, $-(CH_2)_3-CO-(CH_2)_6-$, $-(CH_2)_3-CO-(CH_2)_7-$, $-(CH_2)_3-CO-(CH_2)_8-$, $-(CH_2)_3-CO-(CH_2)_9-$, $-(CH_2)_3-CO-(CH_2)_{10}-$, $-(CH_2)_3-CO-(CH_2)_{11}-$, $-(CH_2)_3-CO-(CH_2)_{12}-$, $-(CH_2)_3-CO-(CH_2)_{13}-$, $-(CH_2)_3-CO-(CH_2)_{14}-$, $-(CH_2)_3-CO-(CH_2)_{15}-$, $-(CH_2)_3-CO-(CH_2)_{16}-$, $-(CH_2)_3-CO-(CH_2)_{17}-$, $-(CH_2)_3-CO-(CH_2)_{18}-$, $-(CH_2)_3-CO-(CH_2)_{19}-$,

$-(CH_2)_3-CO-(CH_2)_{20}-$, $-(CH_2)_3-O-CO-(CH_2)_2-$, $-(CH_2)_3-O-CO-(CH_2)_3-$, $-(CH_2)_3-O-CO-(CH_2)_4-$, $-(CH_2)_3-O-CO-(CH_2)_5-$, $-(CH_2)_3-O-CO-(CH_2)_6-$, $-(CH_2)_3-O-CO-(CH_2)_7-$, $-(CH_2)_3-O-CO-(CH_2)_8-$, $-(CH_2)_3-O-CO-(CH_2)_9-$, $-(CH_2)_3-O-CO-(CH_2)_{10}-$, $-(CH_2)_3-O-CO-(CH_2)_{11}-$, $-(CH_2)_3-O-CO-(CH_2)_{12}-$, $-(CH_2)_3-O-CO-(CH_2)_{13}-$, $-(CH_2)_3-O-CO-(CH_2)_{14}-$, $-(CH_2)_3-O-CO-(CH_2)_{15}-$, $-(CH_2)_3-O-CO-(CH_2)_{16}-$, $-(CH_2)_3-O-CO-(CH_2)_{17}-$, $-(CH_2)_3-O-CO-(CH_2)_{18}-$, $-(CH_2)_3-O-CO-(CH_2)_{19}-$, $-(CH_2)_3-O-CO-(CH_2)_{20}-$, $-(CH_2)_3-CO-O-(CH_2)_2-$, $-(CH_2)_3-CO-O-(CH_2)_3-$, $-(CH_2)_3-CO-O-(CH_2)_4-$, $-(CH_2)_3-CO-O-(CH_2)_5-$, $-(CH_2)_3-CO-O-(CH_2)_6-$, $-(CH_2)_3-CO-O-(CH_2)_7-$, $-(CH_2)_3-CO-O-(CH_2)_8-$, $-(CH_2)_3-CO-O-(CH_2)_9-$, $-(CH_2)_3-CO-O-(CH_2)_{10}-$, $-(CH_2)_3-CO-O-(CH_2)_{11}-$, $-(CH_2)_3-CO-O-(CH_2)_{12}-$, $-(CH_2)_3-CO-O-(CH_2)_{13}-$, $-(CH_2)_3-CO-O-(CH_2)_{14}-$, $-(CH_2)_3-CO-O-(CH_2)_{15}-$, $-(CH_2)_3-CO-O-(CH_2)_{16}-$, $-(CH_2)_3-CO-O-(CH_2)17-$, $-(CH_2)_3-CO-O-(CH_2)_{18}-$, $-(CH_2)_3-CO-O-(CH_2)_{19}-$ oder $-(CH_2)_3-CO-O-(CH_2)_{20}-$.

Als erfindungsgemäße Verbindungen sind z. B. im einzelnen zu nennen:

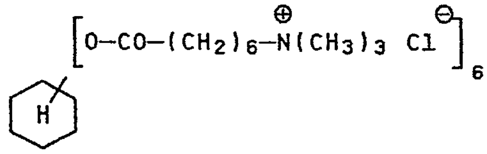
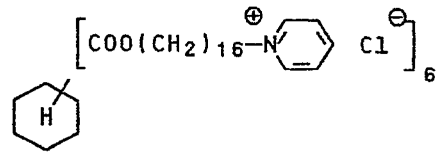
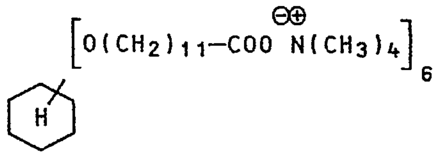
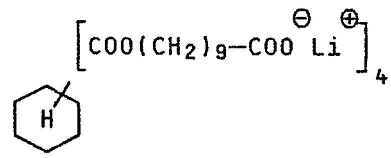
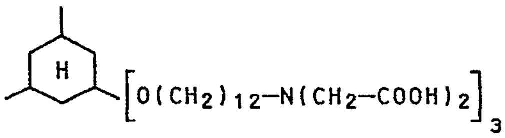
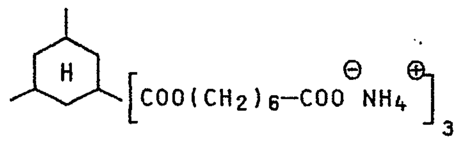
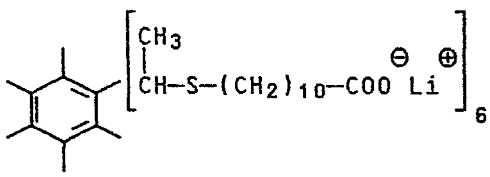
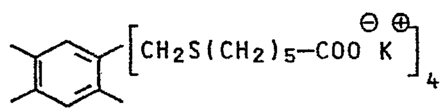
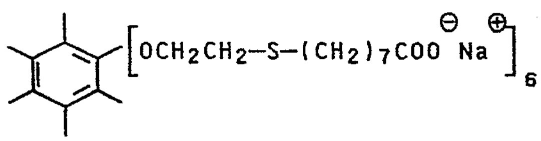
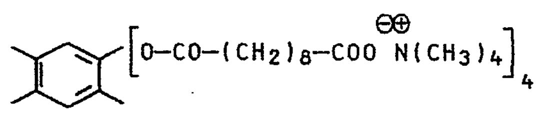
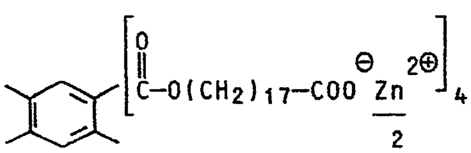
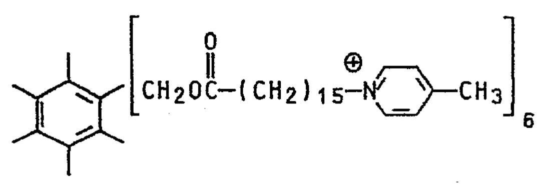
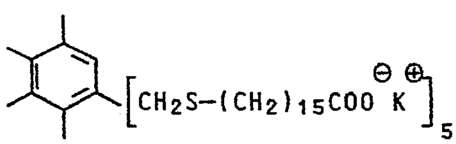
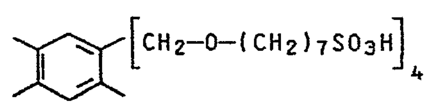
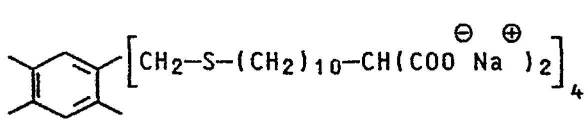
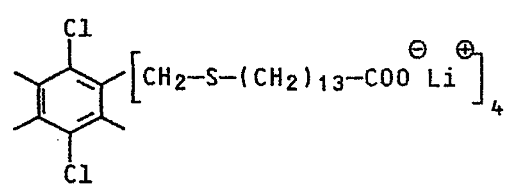

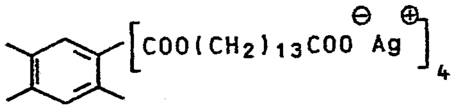

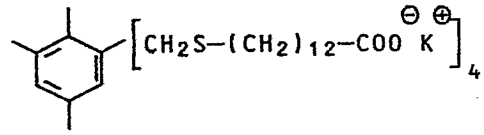

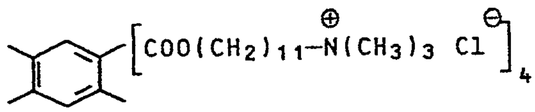

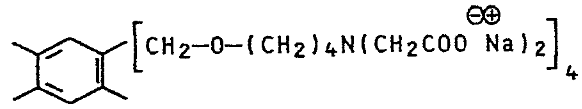

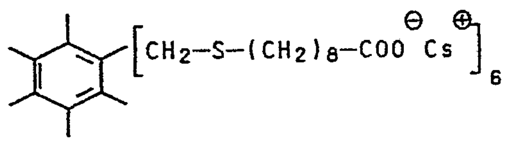

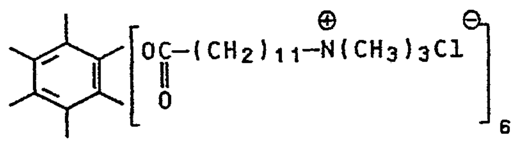

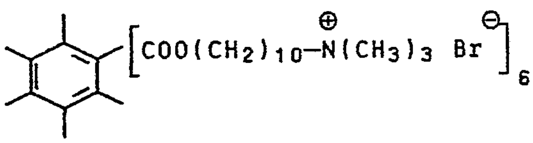

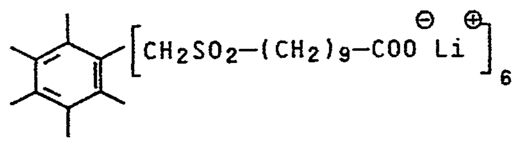

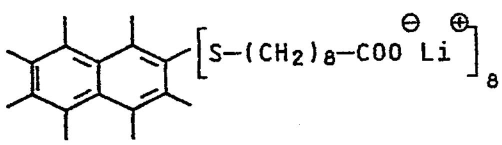

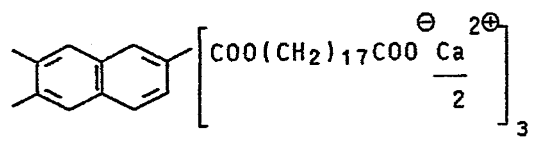

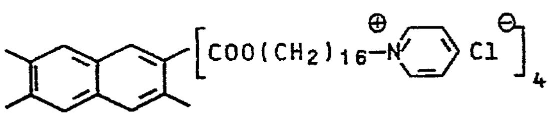

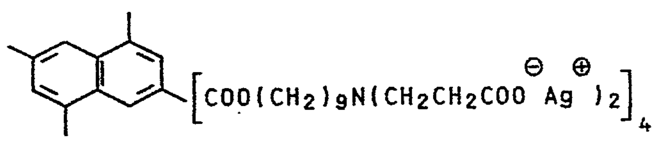

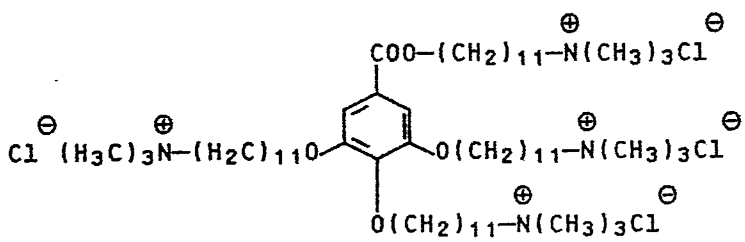

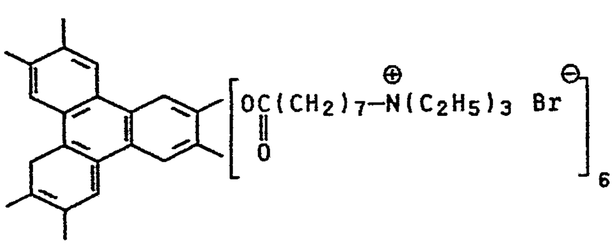

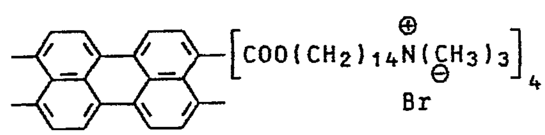

Die erfindungsgemäßen Verbindungen können nach an sich bekannten Verfahren hergestellt werden. Einzelheiten der Herstellung können den Beispielen entnommen werden. Die neuen Verbindungen können in sehr verschiedener Weise angewendet werden. Die Anwendung ergibt sich zum einen aus der Eigenschaft, unterhalb einer bestimmten Konzentration Zylindermicellen zu bilden, zum anderen aus der Eigenschaft, oberhalb einer bestimmten Konzentration in wässriger oder wässrig/alkoholischer Lösung lyotrop nematische Phasen auszubilden. Dementsprechend sind die erfindungsgemäßen Verbindungen beispielsweise als Komplexbildner, Waschrohstoffe, Tenside, Netzmittel, Schmierstoffadditive oder Strömungsbeschleuniger geeignet.

Die erfindungsgemäßen Verbindungen und deren Herstellung sollen durch die folgenden Beispiele näher erläutert werden. Die Teile und Prozentangaben beziehen sich, sofern nicht anders vermerkt, auf das Gewicht.

Beispiel 1

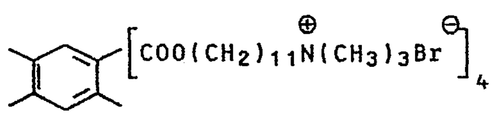

a) 34 g (0,16 mol) Pyromellitsäureanhydrid, 160 g (0,64 mol) 4-Bromundecanol und eine katalytische Menge 4-Toluolsulfonsäure werden in Toluol gelöst und unter Rückfluß am Wasserabscheider gekocht. Wenn kein Wasser mehr abgeschieden wird, wird das Toluol unter vermindertem Druck abdestilliert. Der Rückstand wird mit Ether aufgenommen, filtriert und die Etherlösung mit 0,1 n Salzsäure, gesättigter $NaHCO_3$-Lösung und Wasser ausgeschüttelt. Nach dem Trocknen mit $MgSO_4$ wird der Ether abdestilliert und das Rohprodukt über Kieselgel säulenchromatographisch gereinigt [Petrolether/Ethylacetat (4:1)].
Ausbeute: 148 g (78 % d. Th.) Fp.: 39 - 40 °C
Elementaranalyse: ber.: C 54,64 H 7,64 Br 26,93
gef.: C 54,85 H 7,67 Br 26,83

b) 67,7 g (0,057 mol) der Verbindung Beispiel 1a) werden mit 27 g (0,46 mol) Trimethylamin in Form der 33 %igen Lösung in Acetonitril bei 50 °C gerührt bis dünnschichtchromatographisch kein Ester mehr nachweisbar

ist. Das Rohprodukt wird durch Zusatz von Ether ausgefällt und abgesaugt. Die Fällung wird mehrfach mit Ethylacetat digeriert und abgesaugt, dann in Wasser gelöst und gefriergetrocknet.

Ausbeute: 43,7 g (54 % d. Th.) Fp.: 132 °C (Zers.)

| | | | | | |
|---|---|---|---|---|---|
| Elementaranalyse: | ber.: | C 55,69 | H 8,92 | N 3,94 | Br 22,45 |
| | gef.: | C 54,7 | H 9,3 | N 3,6 | Br 22,2 |

Kritische Micellkonzentration: 1,28-Gew.-% in Wasser

Beispiel 2

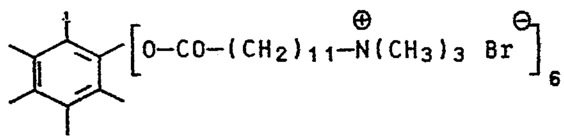

a) 26,1 g (0,15 mol) Hexahydroxybenzol werden mit 296 g (1,08 mol) 11-Bromundecansäurechlorid unter Rühren auf 150 °C erhitzt. Nach vollendeter Reaktion wird die Mischung abgekühlt und in Wasser eingegossen. Der Niederschlag wird abgetrennt und mehrfach mit Ether digeriert bis der Rückstand dünnschichtchromatographisch einheitlich ist und anschließend getrocknet.
Ausbeute: 45 g (17 % d. Th.)

b) 26,1 g (0,015 mol) Ester aus a) werden mit 10,6 g (0,18 mol) Trimethylamin (in Form der 33 %igen Lösung in Ethanol) in Acetonitril unter Rühren solange bei 60 °C gehalten, bis dünnschichtchromatographisch kein Ester mehr nachweisbar ist. Das Rohprodukt wird durch Zusatz von Ether ausgefällt und abgesaugt. Das Filtergut wird mehrfach mit Ethylacetat digeriert, abgesaugt, in Wasser gelöst und gefriergetrocknet.
Ausbeute: 15,7 g (50 % d. Th.)

| | | | | | |
|---|---|---|---|---|---|
| Elementaranalyse: | ber.: | C 53,79 | H 8,72 | N 4,18 | Br 23,85 |
| | gef.: | C 52,9 | H 9,8 | N 4,1 | Br 25,8 |

Beispiel 3

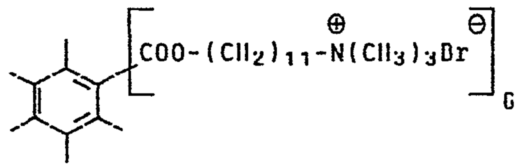

a) Mellithsäurehexamethylester (1 Mol) und 11-Brom-undecanol-(1) (9 Mol) wurden in Chloroform nach Zusatz einer katalytischen Menge konz. $H_2SO_4$ solange erwärmt bis kein $CHCl_3$/Methanol Azeotrop mehr gebildet wurde. Das Lösungsmittel wurde unter vermindertem Druck abgezogen und der Rückstand, wie im Beispiel 1 a) angegeben, aufgearbeitet. Die chromatographische Reinigung erfolgte mit Petrolether/Ethylacetat [6:1 Volumen].

b) Der Hexabromester aus Beispiel 3 a) wurde analog Beispiel 1 b) mit Trimethylamin in Acetonitril umgesetzt und gereinigt.

Elementaranalyse: $C_{96}$ $H_{186}$ $O_{12}$ $N_6$ $Br_6$ M. 2096

| | | | | |
|---|---|---|---|---|
| ber. | C 55,0 | H 8,9 | N 4,0 | Br 22,9 % |
| gef. | C 48,8 | H 8,9 | N 4,5 | Br 27,4 % |

Da die amphiphile Verbindung stark hygroskopisch und wegen ihres hohen Halogengehaltes schwierig zu verbrennen ist, sind die Werte der Elementaranalyse nicht richtig. Es wurden daher die Verhältnisse C/N, C/Br und Br/N ermittelt:

| Verhältnisse | C/N | C/Br | Br/N |
|---|---|---|---|
| ber. | 13,7 | 2,4 | 5,7 |
| gefunden | 10,8 | 1,8 | 6,6. |

Beispiel 4

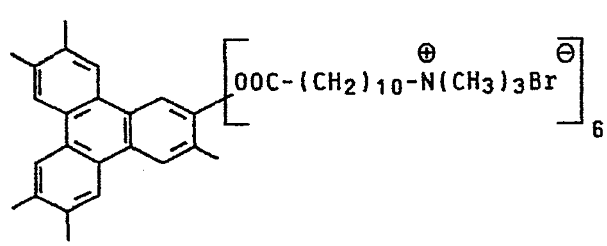

a) Hexahydroxytriphenylen (1 Mol) wurde mit 11-Brom-undecansäurechlorid (7,5 Mol) unter Rühren auf 150°C erwärmt. Nach Beendigung der Reaktion wurde das Reaktionsprodukt über Silicagel chromatographiert. Eluent: $CH_2Cl_2$.

IR-Absorption (Phenylester) C = O 1760 $cm^{-1}$
Elemetaranalyse: $C_{84}$ $H_{126}$ $O_{12}$ $Br_6$ M. 1807,4

| | | | |
|---|---|---|---|
| ber. | C 55,8 | H 7,0 | Br 26,5 % |
| gef. | C 54,3 | H 7,5 | Br 25,1 % |

b) Der Hexabromester aus Beispiel 4 a) wurde analog Beispiel 2 b) mit Trimethylamin umgesetzt und gereinigt.

Elementaranalyse: $C_{102}$ $H_{180}$ $O_{12}$ $N_6$ $Br_6$ M. 2162

| | | | | |
|---|---|---|---|---|
| ber. | C 56,7 | H 8,4 | N 3,9 | Br 22,2 % |
| gef. | C 51,5 | H 9,7 | N 3,8 % | |

Da die Verbindung stark hygroskopisch und wegen des hohen Bromgehaltes schwierig zu verbrennen ist, sind die Werte der Elementaranalyse nicht zutreffend. Es wurde daher das Verhältnis C/N ermittelt:

C/N ber. 14,6, gef. 13,7.

Beispiel 5

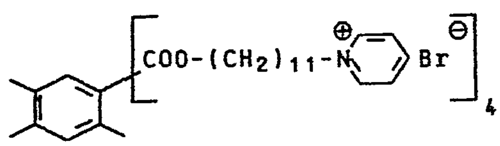

Der nach Beispiel 1 a) erhaltene Tetrabromester wurde analog Beispiel 1 b) quaterniert, wobei Pyridin (100 % Überschuß) anstelle von Trimethylamin angewendet wurde. Die Reinigung erfolgte wie beim Beispiel 1 b) angegeben.

Elementaranalyse: $C_{74}$ $H_{110}$ $O_8$ $N_4$ $Br_4$ M. 1503,4

| | | | | |
|---|---|---|---|---|
| ber. | C 59,1 | H 7,4 | N 3,7 | Br 21,3 % |
| gef. | C 56,1 | H 7,7 | N 3,3 | Br 19,3 % |

Die Verbindung ist stark hygroskopisch und wegen ihres hohen Bromgehaltes schwierig zu verbrennen, weshalb die Werte der Elementaranalyse nicht zutreffend sind. Es wurden daher die Verhältnisse C/N, C/Br und Br/N ermittelt:

| Verhältnisse | C/N | C/Br | Br/N |
|---|---|---|---|
| ber. | 15,9 | 2,8 | 5,7 |
| gef. | 17,0 | 2,9 | 5,8. |

Beispiel 6

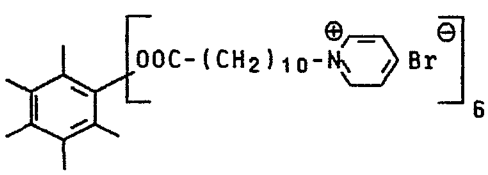

Der nach Beispiel 2 a) erhaltene Hexaester wurde analog Beispiel 2 b) quaterniert, wobei anstelle von Trimethylamin Pyridin (100 % Überschuß) verwendet wurde.

Die Reinigung erfolgte durch Digerieren mit Ethylacetat entsprechend Beispiel 2 a).

Elementaranalyse: $C_{112}$ $H_{150}$ $O_{12}$ $N_6$ $Br_6$ M. 2131,8

| | | | | |
|---|---|---|---|---|
| ber. | C 57,5 | H 7,1 | N 3,9 | Br 22,5 % |
| gef. | C 54,3 | H 7,1 | N 3,6 % | |

Die Verbindung ist stark hygroskopisch und wegen ihres hohen Bromgehaltes schwierig zu verbrennen, weshalb die Werte der Elementaranalyse nicht zutreffend sind. Es wurde daher das Verhältnis C/N ermittelt.

Verhältnis C/N ber. 15,6, gef. 15,2.

Beispiel 7

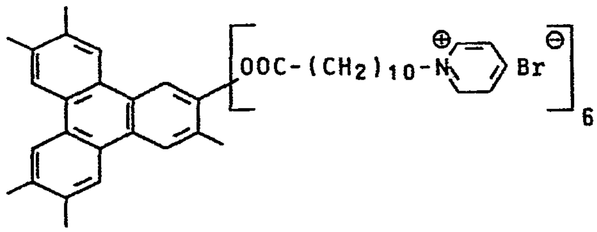

Der nach Beispiel 4 a) erhaltene Ester wurde analog Beispiel 2 b) mit Pyridin (100 % Überschuß) anstelle von Trimethylamin umgesetzt und das Salz wie in Beispiel 2 b) gereinigt.

Elementaranalyse: $C_{114}$ $H_{156}$ $O_{12}$ $N_6$ $Br_6$ M. 2282

| | | | | |
|---|---|---|---|---|
| ber. | C 60,0 | H 6,9 | N 3,7 | Br 21,0 % |
| gef. | C 57,9 | H 8,5 | N 3,0 | Br 19,4 % |

Die Verbindung ist stark hygroskopisch und wegen ihres hohen Bromgehaltes schwierig zu verbrennen, weshalb die Werte der Elementaranalyse nicht zutreffend sind. Es wurden daher die Verhältnisse C/N, C/Br und Br/N ermittelt:

| Verhältnisse | C/N | C/Br | Br/N |
|---|---|---|---|
| ber. | 16,3 | 2,9 | 5,7 |
| gef. | 19,6 | 3,0 | 6,5. |

Patentansprüche

1. Amphiphile flüssigkristalline Verbindungen der allgemeinen Formel

$$D\left[-Z-A-K\right]_i ,$$

in der

D ein Cyclohexanring oder ein planares aromatisches System,

Z eine direkte Bindung oder ein Rest der Formel $-O-$, $-S-$, $-SO_2-$, $-CO-$, $-O-CO-$ oder $-CO-O-$,

A gegebenenfalls durch einen Rest Z unterbrochenes Alkylen und

K ein anionischer oder kationischer Rest sind, wobei

i die Zahlen 4, 5 oder 6 bedeutet, wenn D ein Benzolrest ist, oder > 2 ist, wenn D für einen Cyclohexanring oder ein planares aromatisches System, ausgenommen des Benzols steht.

2. Amphiphile Verbindungen gemäß Anspruch 1, dadurch gekennzeichnet, daß K für

$-SO_3^{\ominus} Me^{\oplus}$, $-N\left[CH_2CH_2SO_3^{\ominus} Me^{\oplus}\right]_2$, $-COO^{\ominus} Me^{\oplus}$, $-N\left[\left[CH_2\right]_r COO^{\ominus} Me^{\oplus}\right]_2$,

$-OCH_2COO^{\ominus} Me^{\oplus}$, $-CH\left[COO^{\ominus} Me^{\oplus}\right]_2$, $-N(R)_3^{\oplus} X^{\ominus}$ oder 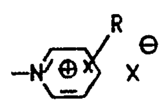 $X^{\ominus}$ steht, wobei r

die Zahl 1 oder 2, R Methyl oder Ethyl, $Me^{\oplus}$ ein Proton, ein Metallion, $NH_4^{\oplus}$ oder $N(CH_3)_4^{\oplus}$ und $X^{\ominus}$ ein Anion bedeuten.

3. Amphiphile Verbindungen gemäß Anspruch 1, dadurch gekennzeichnet, daß D für einen i-wertigen Rest des Benzols steht.

4. Amphiphile Verbindungen gemäß Anspruch 1, dadurch gekennzeichnet, daß D für einen i-wertigen Rest des Cyclohexans steht.

5. Amphiphile Verbindungen gemäß den Ansprüchen 1, 2, 3 oder 4, dadurch gekennzeichnet, daß Z für $-O-$, $-S-$, $-CO-O-$ oder $-O-CO-$ steht.

6. Amphiphile Verbindungen gemäß den Ansprüchen 1, 2, 3, 4 oder 5, dadurch gekennzeichnet, daß A für $[CH_2]_n$ mit n = 6 bis 20 steht.

7. Amphiphile Verbindungen gemäß Anspruch 1, dadurch gekennzeichnet, daß -Z- für eine direkte Bindung und -A- für einen mindestens sechsgliedrigen Rest der Formel $-(CH_2)_p-Z^1-(CH_2)_q-$ steht, wobei $Z^1$ -O-, -S-, -O-CO- oder -CO-O-, und p die Zahlen 1, 2 oder 3 und q die Zahlen 2 bis 20 bedeuten.